(19) **Europäisches Patentamt**

**European Patent Office**

**Office européen des brevets**

(11) Veröffentlichungsnummer: **0 273 121 B1**

(12) **EUROPÄISCHE PATENTSCHRIFT**

(45) Veröffentlichungstag der Patentschrift: **13.05.92**

(21) Anmeldenummer: **87115060.3**

(22) Anmeldetag: **15.10.87**

(51) Int. Cl.⁵: **A61K 37/02**, A61K 35/14

(54) **Oligopeptide aus Rinderblut.**

(30) Priorität: **31.12.86 DE 3644805**

(43) Veröffentlichungstag der Anmeldung:
**06.07.88 Patentblatt 88/27**

(45) Bekanntmachung des Hinweises auf die
Patenterteilung:
**13.05.92 Patentblatt 92/20**

(84) Benannte Vertragsstaaten:
**AT BE CH ES FR GB GR IT LI LU NL SE**

(56) Entgegenhaltungen:
**EP-A- 0 106 309**
**DE-B- 1 076 888**
**FR-A- 2 582 515**

(73) Patentinhaber: **Elkapharm AG**
**Murbacherstrasse 20**
**CH-6005 Luzern(CH)**

(72) Erfinder: **Der Erfinder hat auf seine Nennung**
**verzichtet**

(74) Vertreter: **Siewers, Gescha, Dr.**
**Rechtsanwälte Dr. Harmsen, Dr. Utescher**
**Dipl.-Chem. Harmsen, Bartholatus Dr. Scha-**
**effer, Dr. Fricke, Wolter Patentanwalt Dr. Sie-**
**wers Adenauerallee 28**
**W-2000 Hamburg 1(DE)**

**Beschreibung**

Die Erfindung betrifft aus Rinderblut gewonnene Oligopeptide, Verfahren zu deren Herstellung und deren Verwendung.

Es ist bereits seit langem bekannt, daß sich aus Rinderblut Wirkstoffe mit atmungssteigerndem bzw. stoffwechselaktivierendem Effekt herstellen lassen. So ist beispielsweise in der DE-PS 10 76 888 ein Verfahren zur Gewinnung solcher atmungsfördernden Wirkstoffe aus Rinderblut beschrieben, bei dem das Blut fermentativ abgebaut oder nach einer fraktionierten Enteiweißung zur Entziehung der höher molekularen Bestandteile einer Dialyse mit Wasser bzw. Alkoholen unterworfen wird, woran anschließend das Dialysat vom Lösungsmittel befreit und schonend getrocknet wird. Die nach diesem Verfahren hergestellten Konzentrate verbessern die Sauerstoffverwertung im menschlichen Organismus und können daher bei allen Krankheiten eingesetzt werden, bei denen ein Sauerstoffdefizit zu beheben ist. In der Bundesrepublik sind Blutdialysate z.B. von den Firmen Byk Gulden und Solco unter den Bezeichnungen "Actihaemyl®" bzw. "Solcoseryl®" im Handel, die bei peripheren und zentralen Durchblutungsstörungen und deren Folgekrankheiten Anwendung finden.

Aus der EP-A-0106309 sind Extrakte aus verschiedenen Ausgangsmaterialien wie Blut, Blutplasma, Milch, Knorpel, Plazenta- und Muskelgewebe, Cerealien, Kartoffeln, Fisch, Eiern, Mikroorganismen oder Malz bekannt, die kurzkettige Peptide enthalten und durchblutungsfördernde Eigenschaften aufweisen. Die Herstellung erfolgt durch Zugabe eines Enzymgemisches zur Ausgangsmateriallösung mit einem Feststoffgehalt von etwa 6-14 %, Einwirkung der Enzyme bei 30-60° C, die Aktivierung der Enzyme, Einengung des Nitrates, sowie Sprühtrocknung desselben sowie Reinigung über die Suspension im Alkohol-Wassergemisch und gegebenenfalls abschließender Dialyse. Das Problem bei diesen Blutdialysaten und auch bei anderen Organextrakten liegt aber immer darin, daß die Zusammensetzung solcher Extrakte sehr stark von den Bedingungen des Herstellungsverfahrens abhängt und daher schwankend sein kann, und daß es bis heute nur in seltenen Fällen möglich ist, Zusammensetzung und Struktur solcher Peptide im einzelnen aufzuklären. Eine Strukturaufklärung würde eine Standardisierung und gegebenenfalls auch die Synthese der wirksamen Verbindungen erlauben. Außerdem muß man davon ausgehen, daß bei der Vielzahl der im Dialysat vorliegenden Peptide den einzelnen Verbindungen unterschiedliche bzw. unterschiedlich starke Wirkungen zukommen können, so daß eine Konzentrierung der am stärksten wirksamen Verbindungen und eine Standardisierung dringend erwünscht sind.

Der Erfindung liegt daher die Aufgabe zugrunde, neue Oligopeptidmischungen aus Rinderblutdialysaten zu entwickeln, da noch ein Bedarf an solchen Präparaten mit reproduzierbarer Zusammensetzung besteht.

Zur Lösung der Aufgabe werden Oligopeptidmischungen aus enteiweißtem Rinderblutdialysat vorgeschlagen, die dadurch gekennzeichnet sind, daß sie bei DC-Auftrennung auf Kieselgel mit Fließmittel n-Butanol, Eisessig und Wasser im Verhältnis 4 : 1 : 1 nach vierstündiger Laufzeit und Detektion mit 0,1%-igem Ninhydrinreagenz für 15 Minuten bei 110°C $R_f$-Werte von 0,0045, 0,196, 0,245, 0,4, 0,44, 0,52, 0,66 und 0,8 aufweisen.

Die Herstellung der Oligopeptide erfolgt aus dem Blut veterinärmedizinisch kontrollierter Rinder, insbesondere dessen Keimzahl vorher überprüft wird. Das Rinderblut wird durch Zusatz der dreifachen Menge keimfreiem destilliertem Wassers verdünnt und der pH dann mit Salzsäure oder Natronlauge auf 7,0 eingestellt. Die Mischung wird auf 40°C erwärmt, mit Pepsin und Papain versetzt und 15 Stunden bei 40°C fermentiert. Zur Enzyminaktivierung wird die Gesamtmischung dann für 30 Minuten auf 90°C erhitzt, filtriert und nach dem Abkühlen auf etwa 20°C mit der doppelten Menge mindestens 95 (V/V) %-igem Ethanols versetzt, gekühlt und mindestens 12 Stunden gelagert. Anschließend wird die Mischung eingeengt und dann erneut mit der vierfachen Gewichtsmenge 95%-igem Ethanols versetzt, 5 Stunden gerührt und mindestens 12 Stunden gekühlt. Abschließend wird die Mischung zur Entfernung des Ethanols eingeengt; die verbleibende wässrige Lösung wird ultrafiltriert durch Membranen mit einer Ausscheidungsgrenze für Molekulargewichte von unter 2.000. Dieses Ultrafiltrat wird dann im Vakuum konzentriert und getrocknet. Es resultiert ein gelbes kristallines hygroskopisches Pulver, dessen 1%-ige wässrige Lösung einen pH von etwa 6 - 7 zeigt. Die Osmolalität der 1%-igen Lösung liegt zwischen 0,07 - 0,08 osm/kg. Eine Lösung dieser Substanz ergibt mit Trichloressigsäure und Sulfosalicylsäure keine Fällung, was darauf hinweist, daß keine freien Aminosäuren in nachweisbarer Menge enthalten sind.

Die so hergestellte Peptidmischung wird dünnschichtchromatografisch identifiziert, wobei als Sorbens Kieselgel und als Laufmittel n-Butanol, Eisessig und Wasser im Verhältnis 4 : 1 : 1 bei einer Laufzeit von 4 Stunden und einer Laufstrecke von 15 cm Kammersättigung verwendet werden. Die Detektion erfolgt mit Ninhydrinreagenz 0,1% bei 15 minütiger Erwärmung auf 110°C. Die Auftragsmenge beträgt 0,1 ml. Die Polypeptidmischung weist $R_f$-Werte im Bereich von 0,0045, 0,196, 0,245, 0,4, 0,44, 0,52, 0,66 und und 0,8 auf.

Das Peptidgemisch ergibtnach 24 stündiger Hydrolyse im Durchschnitt folgenden Gehalt an Aminosäuren (Angabe in mmol/g):

| Asparaginsäure | 280 | Methionin | 71,0 |
|---|---|---|---|
| Threonin | 270 | Isoleucin | 42,5 |
| Serin | 300 | Leucin | 540 |
| Glutaminsäure | 240 | Tyrosin | 120 |
| Prolin | 280 | Phenylalanin | 250 |
| Glycin | 340 | Ornithin | 15 |
| Alanin | 570 | Lysin | 285 |
| Cystin | 9,0 | Histidin | 240 |
| Valin | 315 | Arginin | 115 |

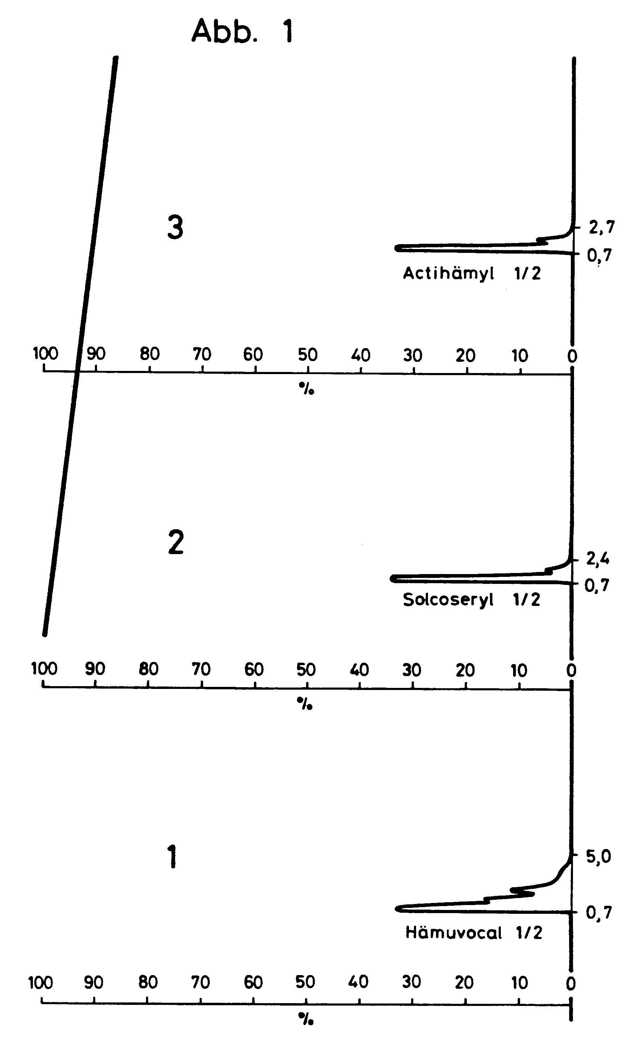

Auch chromatografisch läßt sich das erfindungsgemäße Produkt von den nach bekannten Verfahren hergestellten Produkten eindeutig unterscheiden. Die FPLC wurde mit einem Gerät der Firma Pharmacia durchgeführt, wobei die Säuren HR 5/5 verwandt wurden, die für Auftrennung im Peptidbereich bis 5000 Dalton besonders geeignet sind. Die Laufbedingungen waren für alle Substanzen identisch, als Puffer wurde PBS pH 7,2 eingesetzt; die Fließgeschwindigkeit betrug 1mm/min. Chromatografiert wurde das erfindungsgemäße Produkt in der Verdünnung 1:2 sowie zum Vergleich die nach bekannten Verfahren hergestellten Handelsprodukte "Solcoseryl" und "Actihämyl" ebenfalls in Verdünnung 1:2. Aus den als Abbildung 1 beigefügten Kurvenbildern lassen sich deutlich die Unterschiede feststellen.

Im Warburg-Test, in dem die Steigerung des Sauerstoffverbrauches von Mäuseleberhomogenat gemessen wird, zeigten die erfindungegemäßen Oligopeptide gegenüber der Kontrolle eine Atmungssteigerung von etwa 250%, was auch deutlich über den Werten der bisher im Handel üblichen Präparate in der Größenordnung von etwa 100 bis 150% liegt.

Die nach dem beschriebenen Verfahren hergestellten getrockneten Peptidkonzentrate können in an sich bekannter Weise zu Arzneimitteln für den oralen oder intraversalen Gebrauch weiterverarbeitet werden. Für den oralen Gebrauch werden in üblicher Weise unter Zusatz von Trägerstoffen und weiteren Hilfsstoffen Dragees hergestellt, da hier von einer größeren Haltbarkeit als bei anderen oralen Zubereitungsformen ausgegangen werden kann. Da es sich bei den Wirkstoffen aber um Peptide handelt, wird die galenische Zubereitung vorzugsweise in Form von Injektionslösungen erfolgen, indem die getrocknete Substanz in an sich bekannter Weise in wässriger Lösung weiterverarbeitet wird. Als Konservierungsmittel für Injektionslösungen dieser Art wird vorzugsweise Benzylalkohol eingesetzt. Die Dosierung liegt vorzugsweise im Bereich zwischen etwa 50 - 200 mg des Wirkstoffgemisches.

Die erfindungsgemäß eingesetzten Peptidmischungen führen zu einer Beschleunigung von Stoffwechselvorgängen, und zwar organunabhängig, sowie zu einer Verbesserung der Mikrozirkulation. Dementsprechend können sie bei peripheren und zentralen Durchblutungsstörungen und insbesondere auch zur Verbesserung der Wundheilung wie z.B. nach Verbrennungswunden oder bei Ulzera und auch zur Erhöhung der Strahlentoleranz von Körperzellen und zur verbesserten Adaption von Hauttransplantaten eingesetzt werden.

Die Erfindung wird im folgenden anhand der Beispiele näher erläutert:

1. Herstellung der Peptidmischung

950 1 Rinderblut werden nach Feststellung einer Keimzahl von unter 23 im Verhältnis 1 : 3 mit keimfreiem destilliertem Wasser verdünnt. Der pH-Wert der Lösung wird bestimmt und auf pH 7 durch Zugabe von entweder 1n Salzsäure oder 1n Natronlauge eingestellt. Das verdünnte Blut wird auf 40°C erhitzt und mit je 9,4 kg Pepsin und Papain und 0,05% Benzylalkohol zur Konservierung unter Rühren versetzt. Nach vollständiger Durchmischung wird nochmals die pH Einstellung auf pH 7 überprüft. Unter kontinuierlichem Rühren wird die Enzymeinwirkung über 15 Stunden fortgeführt. Danach erfolgt eine erneute Kontrolle des pH-Wertes. Zur Inaktivierung der Enzyme wird dann die Lösung auf 90°C erhitzt und 30 Minuten bei dieser Temperatur gehalten. Die noch heiße Lösung wird klarfiltriert.

Nach Ermittlung des Trockenrückstandes wird die Lösung entweder durch Einengen oder durch Verdünnen mit keimfreiem destilliertem Wasser so eingestellt, daß auf 1 kg Trockensubstanz 10 l Wasser kommen. Die daraus resultierende 800 l Lösung wird innerhalb einer Stunde unter ständigem Rühren mit 1.600 l mindestens 95 (V/V)%-igem Ethanols versetzt und 5 Stunden nei 20°C gerührt. Die Lösung wird über Nacht in den Kühlraum bei 4°C gelagert. Am darauffolgenden Tag wird die Lösung unter Druck von 0,98-1,46 bar (1-1,5 at) klarfiltriert.

Die alkoholische Lösung wird in einem geeigneten Vakuumverdampfer bei 40°C auf etwa 160 l eingeengt, dann wird der pH-Wert wieder auf pH 7 eingestellt. Die Lösung wird gewogen und innerhalb einer Stunde bei 20°C unter Rühren mit 4 Gewichtsteilen 95%-igem Ethanols versetzt und weitere 5 Stunden gerührt. Nach einer Standzeit im Kühlraum bei 4°C von mindestens 12 Stunden wird die Lösung unter Druck von 0,98-14,6 bar (1-1,5 at) erneut klarfiltriert. Der Alkohol wird anschließend am Verdampfer abgezogen. Zur Ultrafiltration wird die resultierende wässrige Lösung unter Druck durch eine Membran an der Ausscheidegrenze für ein Molekulargewicht von unter 2.000 gepresst. Die wässrige Lösung des Ultrafiltrates wird unter Vakuum aufkonzentriert und getrocknet. Die verbleibende Restfeuchte wird im Trockenschrank aus dem Konzentrat entfernt.

Der Extrakt ist ein gelblicheskristallines hygroskopisches Pulver. Die Ausbeute an ultrafiltrierter Trockensubstanz beträgt etwa 28 kg.

## 2. Herstellung einer Injektionslösung

8.800 kg des ethanolischem Extraktes entsprechend Beispiel 1 und 2.200 kg Benzylakolhol werden unter Rühren in 212.630 kg sterilem Wasser für Injektionszwecke unter Rühren gelöst. Der pH-Wert wird auf pH 5,8 eingestellt. Abschließend wird über ein Cellulosenitratfilter mit der Porengröße 0,2 $\mu$ sterilfiltriert und in sterile Ampullen unter Stickstoffschutz abgefüllt und abgeschmolzen.

## 3. Identifizierung der Peptidmischung

Die Identifizierung wird dünnschichtchromatografisch auf Kieselgel 60 ohne Fluoreszenzindikator der Firma Merck mit Laufmittel n-Butanol, Eisessig und Wasser im Verhältnis 4 : 1 : 1 durchgeführt. Die Laufzeit beträgt 4 Stunden; die Auftragsmenge 1 ml. Die Anfärbung erfolgt mit Ninhydrinsprühreagenz 0,1%-ig, Entwicklung 15 Minuten bei 110°C. Das entwickelte Chromatogramm zeigt folgende $R_f$-Werte: 0,0045 , 0,196 , 0,244 , 0,40 , 0,44 , 0,52 , 0,66 und 0,8.

## Patentansprüche

**1.** Oligopeptidmischungen aus enteiweißtem Rinderblutdialysat, dadurch gekennzeichnet, daß sie bei DC-Auftrennung auf Kieselgel mit Fließmittel n-Butanol, Eisessig und Wasser im Verhältnis 4:1:1 nach 4 stündiger Laufzeit und Detektion mit 0,1%-iger Ninhydrinlösung für 15 Minuten bei 110°C $R_f$-Werte von 0,0045, 0,196 , 0,244 , 0,40 , 0,44 , 0,52 , 0,66 und 0,8 aufweisen.

**2.** Oligopeptidmischung nach Anspruch 1, dadurch gekennzeichnet, daß sie nach 24 Stunden Hydrolyse einen Gesamtgehalt an Aminosäuren in mmol/g von

| Asparaginsäure | 280 | Methionin | 71,0 |
|---|---|---|---|
| Threonin | 270 | Isoleucin | 42,5 |
| Serin | 300 | Leucin | 540 |
| Glutaminsäure | 240 | Tyrosin | 120 |
| Prolin | 280 | Phenylalanin | 250 |
| Glycin | 340 | Ornithin | 15 |
| Alanin | 570 | Lysin | 285 |
| Valin | 315 | Histidin | 240 |
| Cystin | 9,0 | Arginin | 115 |

aufweist.

**3.** Verfahren zur Herstellung einer Peptidmischung nach Anspruch 1 und 2 , dadurch gekennzeichnet, daß Rinderblut nach dem Verdünnen mit der dreifachen Wassermenge auf 40°C erwärmt, mit Pepsin und Papain 15 Stunden bei 40°C fermentiert, zur Enzyminaktivierung für 30 Minuten auf 90°C erhitzt, filtriert, bei etwa 20°C mit der doppelten Menge mindestens 95 (V/V)%-igem Ethanols versetzt, gekühlt und mindestens 12 Stunden gelagert, eingeengt und erneut mit der vierfachen Gewichtsmenge Ethanols 95 (V/V)% versetzt, gerührt, mindestens 12 Stunden gekühlt, eingeengt und ultrafiltriert wird.

**Claims**

1. Oligopeptide mixtures from deproteinized bovine blood dialysate characterized in that they have $R_f$ values of 0.0045, 0.196, 0.244, 0.40, 0.44, 0.52, 0.66 and 0.8 under a thin-layer chromatographic separation on silica gel using a mixture of n-butanol, glacial acetic acid and water in a ratio of 4:1:1 as a developing solvent, after a running time of four hours and detection with a 0.1 % ninhydrin reagent while heating for 15 minutes to 110°C.

2. An oligopeptide mixture according to claim 1, characterized in that it has a total amino acid content in mmoles/g after 24 hours of

| asparaginic acid | 280 | methionine | 71.0 |
|---|---|---|---|
| threonine | 270 | isoleucine | 42.5 |
| serine | 300 | leucine | 540 |
| glutaminic acid | 240 | tyrosine | 120 |
| proline | 280 | phenyl alanine | 250 |
| glycine | 340 | ornithine | 15 |
| alanine | 570 | lysine | 285 |
| valine | 315 | histidine | 240 |
| cystine | 9.0 | arginine | 115. |

3. A method of preparing a peptide mixture according to claims 1 and 2, characterized in that after dilution of the bovine blood by the addition of a threefold amount of water the mixture is heated to 40°C, is fermented with pepsin and papain for 15 hours at 40°C, is heated to 90°C for 30 minutes inactivate the enzymes, is mixed with the double amount of at least 95(V/V)% ethanol at about 20°C, is cooled and stored for at least 12 hours, is concentrated and mixed once again with a fourfold amount by weight of 95(V/V)% ethanol, is agitated, cooled for at least 12 hours, concentrated and ultrafiltered.

**Revendications**

1. Mélanges d'oligopeptides à partir d'un dialysat de sang de boeuf déprotéinisé, caractérisé en ce qu'ils présentent des valeurs due facteur de rétention de 0,0045, 0,196, 0,244, 0,40, 0,52, 0,66 et 0,8 sous séparation par chromatographie en couche mince sur du gel de silice en utilisant le solvant n-butanol, de l'acide acétique glacial et de l'eau dans la proportion de 4 : 1 : 1 après une période d'élution de quatre heures et détection avec une solution à base de 0,1 % de ninhydrine pour 15 minutes par 110°C.

2. Mélange d'oligopeptides selon la revendication 1, caractérisé en ce qu'il présente après 24 heures d'hydrolyse une teneur totale en aminoacides en mmol/g de

| acide aspartique | 280 | méthionine | 71,0 |
|---|---|---|---|
| thréonine | 270 | isoleucine | 42,5 |
| sérine | 300 | leucine | 540 |
| acide glutamique | 240 | tyrosine | 120 |
| proline | 280 | phénylalanine | 250 |
| glycine | 340 | ornithine | 15 |
| alanine | 570 | lysine | 285 |
| valine | 315 | histidine | 240 |
| cystine | 9,0 | arginine | 115. |

**3.** Procédé de préparation d'un mélange de peptides selon les revendications 1 et 2, caractérisé en ce que le sang de boeuf, après addition d'une triple quantité d'eau, est porté à une température de 40 °C, est fait fermenté avec pepsine et papaine pendant 15 heures, est chauffé à une température de 90°C pendant 30 minutes pour désactiver les enzymes, est filtré, est mélangé avec la double quantité d'éthanol avec au moins 95 pourcent volumétrique par environ 20°C, est refroidi et fait reposer pendant 12 heures, est concentré et encore une fois mélangé avec la quadruple quantité en poids d'éthanol 95 (V/V) %, est agité, est refroidi pendant au moins 12 heures, est concentré et ultrafiltré.

# Abb. 1

**3**

2,7
0,7

Actihämyl 1/2

100  90  80  70  60  50  40  30  20  10  0

%

**2**

2,4
0,7

Solcoseryl 1/2

100  90  80  70  60  50  40  30  20  10  0

%

**1**

5,0

0,7

Hämuvocal 1/2

100  90  80  70  60  50  40  30  20  10  0

%